# EUROPÄISCHE PATENTSCHRIFT

(11) **EP 1 844 740 B1**
(45) Veröffentlichungstag und Bekanntmachung des Hinweises auf die Patenterteilung: **19.12.2012**
(21) Anmeldenummer: 07005177.6
(22) Anmeldetag: 13.03.2007
(51) Int. Cl.: A61F 2/90

(54) **Selbstexpandierender Stent mit Federstruktur**
Self-expanding stent with spring structure
Stent auto-extensible doté d'une structure à ressort

(30) Priorität: 11.04.2006 DE 102006017028
(43) Veröffentlichungstag der Anmeldung: 17.10.2007
(73) Patentinhaber: Admedes Schuessler GmbH, 75179 Pforzheim (DE)
(72) Erfinder: Schuessler, Andreas, Dr., 76327 Pfinztal (DE); Steiner, Ralf, 75181 Pforzheim (DE)
(74) Vertreter: Müller-Boré & Partner Patentanwälte

(56) Entgegenhaltungen:
- WO-A-98/20810
- WO-A2-02/074198
- GB-A- 2 418 362
- US-A1- 2004 153 140

## Beschreibung

Die vorliegende Erfindung betrifft einen Stent zum Implantieren in einen lebenden Körper und ein Verfahren zum Herstellen desselben.

Körperimplantate bzw. Stents dieser Art schützen Kanäle lebender Körper wie beispielsweise Blutgefäße, Speiseröhre, Harnröhre oder Nierengänge durch Einführen des Stents und Expandieren des Stents im Inneren des Körperkanals. Auf diese Weise kann ein Kollabieren oder Verschließen des jeweiligen Körperkanals verhindert werden. Darüber hinaus wird ein Stent beispielsweise für interzerebrale Gefäßaussackungen, sogenannte Aneurismen eingesetzt, die die häufigste Ursache für nicht-traumatische Subarachnoidalblutungen sind. Ihre Inzidenz liegt in der Gesamtbevölkerung bei 1%, nach Autopsiestudien sogar bis zu 9%. Pathomorphologisch sind interzerebrale Aneurismen in der Regel echte, sacurale Aneurismen, die meist in Gefäßaufzweigungen lokalisiert sind (vgl. beispielsweise Schumacher M. "Diagnostic work-up in cerebral aneurysms" in Nakstadt PHj (ed): "cerebral aneurysms", pp 13-24, Bologna: Centauro (2000).

Darüber hinaus können derartige Körperimplantate bzw. Stents als Träger von Medikamenten dienen, um eine lokale Therapie innerhalb des Körperkanals zu ermöglichen. Diese Stents werden in einem kollabierten Zustand in einen Körperkanal eingeführt und nach Anordnung des Stents in dem Körperkanal expandiert. Die Stents bestehen üblicherweise aus rostfreiem Edelstahl oder einer Kobalt-Chrom-Tantal-Legierung. Die Stents werden bevorzugt durch eine Aufweitungseinrichtung wie beispielsweise einem Ballonkatheter in den Körperkanal eingeführt und dort aufgeweitet.

Die Stents können jedoch auch aus anderen Werkstoffen wie beispielsweise Polymeren, selbst abbaubaren Werkstoffen wie beispielsweise Milchsäurewerkstoffen bzw. Derivaten sowie aus Nitinol (Nickel-Titan-Legierungen) und/oder aus anderen selbst expandierbaren Werkstoffen wie beispielsweise sogenannten Formgedächtnis-Werkstoffen bestehen.

Die Stegstruktur solcher Stents ist aus einer Vielzahl von Stegen gebildet, die jeweils an Knotenabschnitten miteinander gekoppelt sind und einzelne nebeneinander angeordnete Zellen begrenzen. Durch Aufweiten der einzelnen Zellen kann die Stegstruktur insgesamt expandiert und bei bestimmten Stents nachfolgend durch Verkleinern der Zellen auch wieder verkleinert werden. Die Stege bilden also Verbindungselemente zwischen den Knotenabschnitten, welche im wesentlichen steif sind und wesentlich zur Stützwirkung eines Stents beitragen.

Damit der Stent an der Kanalwand anliegt, muss er radial im Kanal expandierbar sein. Darüber hinaus muss der Stent im expandierten Zustand seiner Stützfunktion gerecht werden.

Wenn ein derartiger Stent zusätzlich zu seiner radialen Expansionsfähigkeit auch in Längsrichtung, d.h. in Richtung einer Stentachse flexibel sein soll, müssen Brücken- und Verbinderabschnitte zwischen den Stentzellen angeordnet werden, die die erforderliche Flexibilität in Längsrichtung des Stents aufweisen, da die Stege als steife Balken ausgebildet sind, die keine oder nur eine sehr geringe Flexibilität in Längsrichtung haben, wie beispielsweise in der DE 103 23 475 A1 beschrieben ist.

US 2004/0153140 A1 offenbart angioplastische Stents mit einer Vielzahl von Segmenten, die paarweise durch Brückenmittel verbunden sind, so dass aufeinanderfolgende Segmente zwecks Biegen des Körpers in beliebiger Richtung relativ zueinander ausgerichtet werden können.

Die Aufgabe der vorliegenden Erfindung besteht somit in der Schaffung eines Körperimplantats, das eine erhöhte Flexibilität insbesondere in Längsrichtung aufweist, so dass auch große Deformationen des Körperimplantats ohne Beschädigung desselben aufgenommen werden können und darüber hinaus das Anliegen der Stentstruktur an einer Körperkanalwandung verbessert ist. Des weiteren soll ein Herstellungsverfahren für ein derartiges Körperimplantat (Stent) geschaffen werden.

Diese Aufgabe wird durch einen Stent gemäß Anspruch 1 und ein Herstellungsverfahren gemäß Anspruch 11 gelöst. Vorteilhafte Weiterbildungen sind in den abhängigen Ansprüchen definiert.

Der Stent zum Einführen oder Implantieren in einen lebenden Körper hat eine Struktur, die zumindest bereichsweise Stege aufweist, um einzelne Stentzellen zu bilden, wobei die Stege zumindest teilweise eine Federstruktur aufweisen, wobei die Federstruktur durch eine Vielzahl von Mikrostegen gebildet ist, die an Knotenpunkten miteinander verbunden sind, wobei die Mikrostege einen kreisförmigen oder elliptischen Querschnitt mit einem Durchmesser von 40 µm bis 200 µm oder einen rechteckigen Querschnitt mit einer Kantenlänge von 40 µm bis 200 µm aufweisen, wobei die Mikrostege eine Steglänge von 100 µm bis 2000 µm und die Stege eine Länge von 1000 µm bis 10000 µm aufweisen, wobei ein Steg 8 bis 15 Mikrostege aufweist, und wobei eine Vielzahl an Reihen von Stegen nebeneinander angeordnet sind und jeweils vier Stege durch ein Zwischenelement verbunden sind, das elastisch verformbar ist. Somit wird die Elastizität bzw. Deformationsfähigkeit des Körperimplantats wesentlich erhöht, da jeder eine Federstruktur aufweisende Steg insbesondere in seiner Längsrichtung gedehnt und gestreckt werden kann. Die Verformbarkeit des Stegs mit der Federstruktur ist jedoch nicht darauf beschränkt. Vielmehr ist auch je nach Konfiguration der Mikrofederstruktur eine Knickung bzw. Biegung des Stegs möglich. Idealerweise wird die Konfiguration dabei mittels einer Finite-Elemente-Methode bestimmt, um die gewünschte Verformbarkeit der Mikrofederstruktur zu erzielen.

Alternativ oder zusätzlich kann der Stent zumindest teilweise flexible Elemente aufweisen, die vorzugsweise auf elastische Weise in der Länge gedehnt bzw. gestreckt und/oder gestaucht werden können und/oder gebogen und/oder tordiert/verdreht werden können. Elastische Weise bedeutet (im Gegensatz zur plastischen Verformung), dass die flexiblen Elemente keine bleibende Verformung erfahren, sondern nach dem Wegnehmen einer Verformungskraft ihre ursprüngliche Gestalt im wesentlichen wieder annehmen, d.h. die Federstruktur bilden. Somit ist das Körperimplantat derart flexibel, dass beispielsweise eine elastische Längendehnung des Körperimplantats um bis zu etwa 50%, vorzugsweise von etwa 20 bis etwa 40 % und am meisten bevorzugt von etwa 30 bis etwa 35 % möglich ist. Vorzugsweise sind die flexiblen Elemente um bis zu etwa 200 %, am meisten bevorzugt bis zu etwa 100% in der Länge flexibel verformbar, und zwar in der Dehnungsrichtung und/oder der Stauchungsrichtung. Vorzugsweise sind diese flexiblen Elemente derart flexibel, dass die Verformung mit sehr geringer Kraft erfolgen kann, d.h. beispielsweise durch einen ballonexpandierten Katheter.

Dabei kann je nach Konfiguration der Mikrofederstruktur der Stege bzw. elastischen Elemente und der Konfiguration der durch die Stege gebildeten Stentzellen eine bevorzugte erhöhte Verformbarkeit des Stents bzw. Körperimplantats erzielt werden. Dies ist insbesondere bei Stents für Beinarterien vorteilhaft, da die Dauerbelastbarkeit durch eine derartige Stentstruktur erheblich gesteigert werden kann ohne dass es zu plastischen Verformungen einzelner Stege oder gar zu Brüchen der Stege kommt.

Vorzugsweise ist die Mikrofederstruktur zumindest teilweise durch flexible Elemente und/oder eine Zick-Zack-Form der Stege und/oder eine Mäander- oder Wellen-Form der Stege gebildet. Die Stege können jedoch auch jede andere Federform haben wie beispielsweise die Form einer Schraubenfeder, Blattfeder, Parabelfeder oder einer Spiralfeder. Insbesondere die Ausbildung der Mikrofeder als Schraubenfeder bietet den Vorteil einer noch höheren Verformbarkeit, während die Ausbildung als Blattfeder den Vorteil einer vereinfachten Federform bietet, die einfach und kostengünstig herstellbar ist.

Erfindungäsgemäß ist die Federstruktur jedes Stegs durch eine Vielzahl von Mikrostegen gebildet. Da sich die Mikrostege zueinander bewegen bzw. verformen können, wird gegenüber der Ausbildung eines Stegs als steifer Balken, wie im Stand der Technik, eine erhöhte Flexibiltät des Stegs erzielt. Als ein Mikrosteg wird dabei ein Steg bezeichnet, der einen Durchmesser bzw. eine Kantenlänge von 40 µm bis 200 µm, vorzugsweise von 70 bis 170 µm und am meisten bevorzugt von 100 bis 160 µm und eine Steglänge L von 100 µm bis 2000 µm, vorzugsweise von 200 bis 1500 µm und am meisten bevorzugt von 400 bis 1000 µm hat.

Erfindunusgemäß sind die Mikrostege an Knotenpunkten miteinander verbunden. Durch eine Biegung der Mikrostege zwischen den Knotenpunkten wird die hohe Verformbarkeit des die Mikrostege aufweisenden Stegs erzielt.

Des weiteren sind bei dem Körperimplantat, vier Stege durch ein Zwischenelement verbunden, das ebenfalls elastisch verformbar gestaltet ist. Hierzu hat das Zwischenelement vorzugsweise eine Zick-Zack-Form oder eine Wellenform.

Des weiteren ist eine Reihe von Stegen durch zick-zack-förmig angeordnete und an jeweiligen Stegenden miteinander verbundene Stege gebildet und es ist eine Vielzahl an Reihen von Stegen nebeneinander angeordnet. Die Reihen der Stege sind dabei entweder entlang der Umfangsrichtung eines rohrförmigen Stents oder entlang der Axialrichtung eines rohrförmigen Stents angeordnet. Es ist jedoch auch möglich die Reihen der Stege diagonal bzw. schrauben- oder schneckenförmig anzuordnen.

Vorzugsweise wechseln sich entlang einer Reihe von Stegen in regelmäßiger Reihenfolge freie Enden der Stege mit durch Zwischenelemente verbundenen Enden von Stegen ab, wobei die Zwischenelemente die verbundenen Enden der Stege mit verbundenen Enden der Stege der nebenliegenden Reihe verbinden. Weiter bevorzugt folgt entlang einer Reihe von Stegen auf zwei freie Enden der Stege jeweils ein durch ein Zwischenelement mit der nebenliegenden Reihe von Stegen verbundenes Ende eines Stegs.

Der Stent wird dabei zumindest teilweise aus Nitinol hergestellt, da Nitinol nach dem Überschreiten einer elastischen Verformung nicht sofort plastisch verformt wird, sondern bis zu 8% superelastisch verformt werden kann.

Des weiteren wird erfindungsgemäß ein Verfahren zum Herstellen eines Stents geschaffen mit den Schritten:
Ausbilden einer Stentstruktur, die zumindest bereichsweise Stege aufweist; und
Ausbilden der Stege zumindest teilweise mit einer Federstruktur, Bilden der Federstruktur durch eine Vielzahl von Mikrostegen, die an Knotenpunkten miteinander verbunden sind, Formen der Mikrostege derart, dass diese einen kreisförmigen oder elliptischen Querschnitt mit einem Durchmesser von 40 µm bis 200 µm oder einen rechteckigen Querschnitt mit einer Kantenlänge von 40 µm bis 200 µm aufweisen, wobei die Mikrostege eine Steglänge von 100 µm bis 2000 µm und die Stege eine Länge von 1000 µm bis 10000 µm aufweisen, wobei ein Steg 8 bis 15 Mikrostege aufweist, und Anordnen einer Vielzahl an Reihen von Stegen nebeneinander und Verbinden von jeweils vier Stegen durch ein Zwischenelement, das elastisch verformbar ist.

Erfindungsgemäß wird die Federstruktur durch eine Vielzahl an Mikrostegen ausgebildet. Weiter bevorzugt wird zumindest eine Reihe von Stegen durch zick-zack-förmig angeordnete und an jeweiligen Stegenden miteinander verbundene Stege ausgebildet. Des weiteren werden bevorzugt zwei nebeneinander angeordnete Reihen von Stegen durch Zwischenelemente miteinander verbunden, indem verbundene Enden von Stegen der einen Reihe mit verbundenen Enden der Stege der nebenliegenden Reihe verbunden werden.

Vorzugsweise wird die Stentstruktur mit den eine Mikrofeder bildenden Stegen und Zwischenelementen durch Laserschneiden oder Erodieren gebildet. Die Mikrofeder des Steges kann jedoch auch durch einen Stanz- oder Laserschweißprozess einfach hergestellt werden, indem sie in der Mantelfläche des Stents ausgebildet bzw. ausgeschnitten wird. Darüber hinaus kann die Stentstruktur durch (Photo)-Ätzen aus Rohr- oder Flachmaterial, durch eine Sputtertechnik (PLD) oder aus einem Draht hergestellt werden.

Nachfolgend werden Ausführungsbeispiele eines erfindungsgemäßen Stents anhand der beigefügten schematischen Zeichnungen näher erläutert. Es zeigt:
Fig. 1 eine Draufsicht auf eine erfindungsgemäße Stentstruktur mit Stegen 11 und Zwischenelementen 21;
Fig. 2 eine vergrößerte Detailansicht der Stegstruktur von Fig. 1;
Fig. 3 eine Draufsicht auf eine erfindungsgemäße Stentstruktur mit Stegen 11 und Zwischenelementen 21.

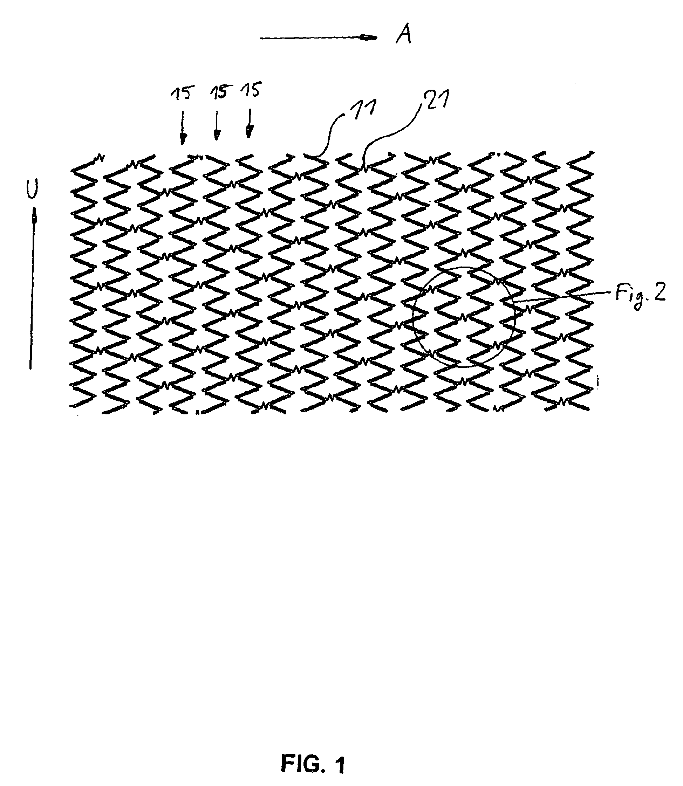

Wie in Fig. 1 gezeigt ist, ist die Stentstruktur durch eine Vielzahl von Stegen bzw. flexiblen Elementen 11 gebildet, die in Reihen 15 angeordnet sind. Gemäß dem in Fig. 1 gezeigten Ausführungsbeispiel sind die Reihen 15 dabei in Umfangsrichtung U des Stents angeordnet. In axialer Richtung A des Stents schließen sich mehrere Reihen 15 an.

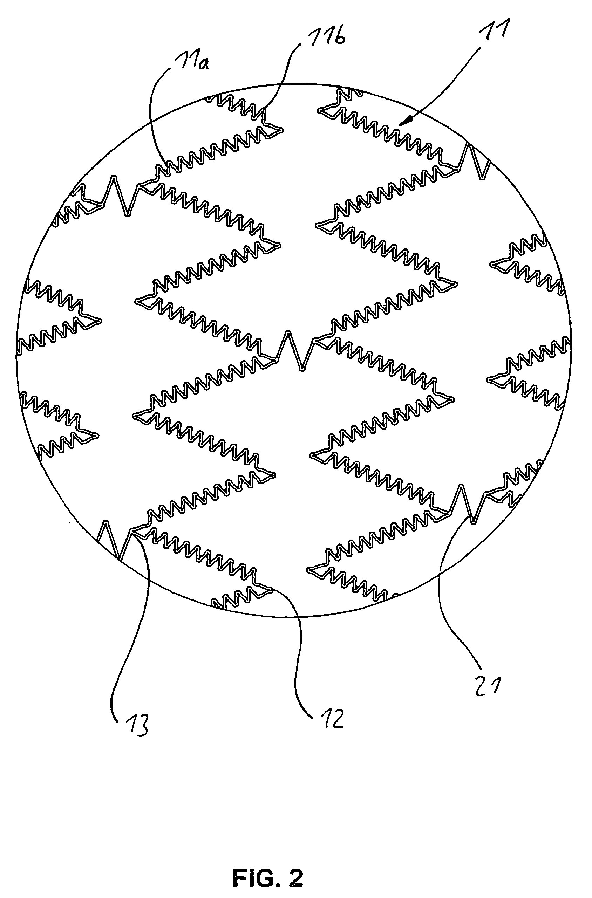

Im Gegensatz zu den Stentstrukturen nach dem Stand der Technik sind die Stege 11 jedoch nicht als steife Balken gebildet, sondern haben eine Mikrostruktur, die besonders elastisch ist, wie in Fig. 2 gezeigt ist. Dies wird dadurch erzielt, dass der Steg 11 aus einer Vielzahl von Mikrostegen 11 a gebildet ist, die an Knotenpunkten 11 b miteinander verbunden sind. Dabei hat ein Steg 11 cirka 3 bis 30 Mikrostege, vorzugsweise 5 bis 20 Mikrostege, noch mehr bevorzugt 8 bis 15 Mikrostege.

Die Mikrostege 11a haben vorzugsweise einen kreisförmigen oder elliptischen Querschnitt oder einen rechteckigen Querschnitt mit einem Durchmesser bzw. einer Kantenlänge von 40 µm bis 200 µm, vorzugsweise von 70 bis 170 µm und am meisten bevorzugt von 100 bis 160 µm und eine Steglänge L von 100 µm bis 2000 µm, vorzugsweise von 200 bis 1500 µm und am meisten bevorzugt von 400 bis 1000 µm. Die Stege 11 haben eine Länge von 1000 µm bis 10000 µm.

Durch Ausbilden der Mikrostege 11a beispielsweise durch Laserschneiden eines aus Nitinol hergestellten Stents erhält jeder Steg 11 eine Mikrofederstruktur, da sich zusätzlich zu dem Durchbiegen des Stegs 11 zwischen seinen Knotenpunkten 12, 13 durch das Durchbiegen der Mikrostege 11a zwischen den Knotenpunkten 11b eine erhöhte Elastizität des Stegs 11 ergibt. Dadurch kann eine Stentstruktur mit höherer Verformungsfähigkeit als nach dem Stand der Technik geschaffen werden.

Diese Stentstruktur eignet sich insbesondere für Stents, die in Beinarterien eingesetzt werden, da diese Stents besonders hohen Belastungen ausgesetzt sind. Durch die erhöhte Elastizität wird einerseits eine plastische Verformung des Stents und andererseits eine Beschädigung des Stents vermieden. Darüber hinaus liegt der erfindungsgemäße Stent aufgrund seiner hohen Elastizität besser an der Wandung des Körperkanals an, so dass die Stützfunktion des Stents verbessert ist.

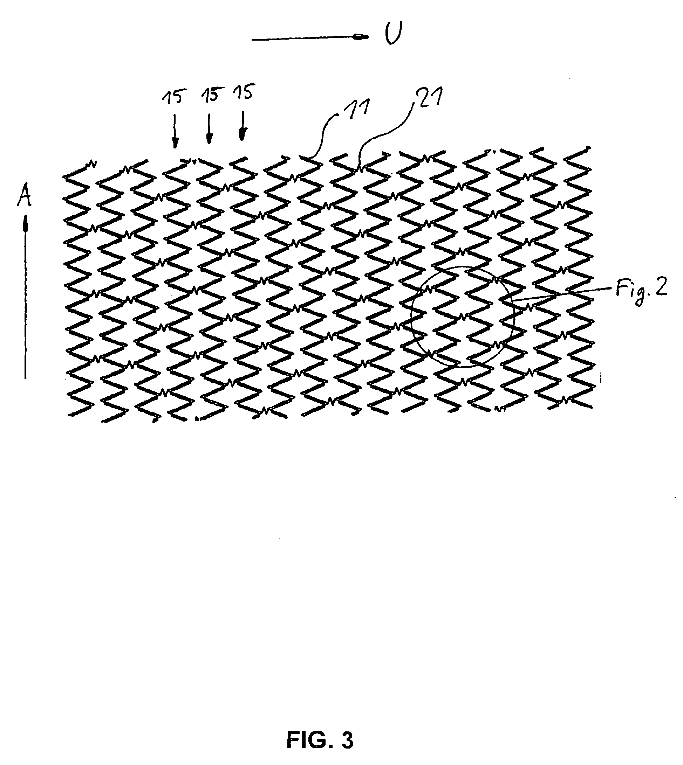

Fig. 3 zeigt ein zweites Ausführungsbeispiel, bei dem die Reihen 15 der Stege 11 entlang der Axialrichtung A des Stents angeordnet sind und in Umfangsrichtung U mehrere Reihen 15 angeordnet sind.

Obwohl es hier nicht gezeigt ist, kann der erfindungsgemäße Stent sowohl entlang der Umfangsrichtung U angeordnete Reihen 15 von Stegen 11 gemäß dem ersten Ausführungsbeispiel als auch in Axialrichtung A des Stents angeordnete Reihen 15 von Stegen 11 aufweisen. Darüber hinaus ist es auch möglich, die Reihen 15 schräg zur Umfangsrichtung U anzuordnen, so dass die Reihen 15 eine Schrauben- oder Schneckenform bilden.

Darüber hinaus sind die Reihen 15 durch Zwischenelemente 21 an verbundenen Enden 13 der Stege 11 miteinander verbunden. Diese Zwischenelemente 21 können ebenfalls als flexible Elemente ausgeführt sein. Dabei hat es sich als vorteilhaft herausgestellt, wenn sich nicht verbundene freie Enden 12 der Stege 11 mit verbundenen Enden 13 abwechseln. Als besonders vorteilhaft hat sich eine Stentstruktur herausgestellt, bei der entlang einer Reihe 15 sich jeweils zwei freie Enden 12 mit einem verbundenen Ende 13 abwechseln. Je nach gewünschter Elastitzität des Stents ist jedoch auch jede andere Konfiguration möglich.

Das Zwischenelement 21 ist dabei vorzugsweise auch elastisch ausgebildet, indem es eine Zick-Zack-Form oder eine Wellen- oder Mänderform aufweist. Die Mikrostege 11a bilden zusammen mit den Knotenpunkten 11 b ebenfalls eine Zick-Zack-Form. Die Mikrostege 11a können, obwohl es hier nicht gezeigt ist, jedoch auch eine Wellen- oder Mänderform aufweisen. Darüber hinaus kann die Mikrofederstruktur auch durch eine Schraubenfederform der Mikrostege gebildet werden. Ebenfalls möglich ist eine Blattfeder oder Spiralfederform.

Es können auch unterschiedliche Kombinationen von Federformen der Mikrostege 11a eingesetzt werden, die eine bevorzugte Elastizität in einer vorgegebenen Richtung bzw. Steifigkeit in einer anderen Richtung aufweist, die vorzugsweise mittels durch eine Finite-Elemente-Methode bestimmt wird. Dabei können auch als steife Balken ausgebildete Stege, wie sie aus dem Stand der Technik bekannt sind, mit erfindungsgemäßen Stegen 11 mit der Mikrofederstruktur innerhalb einer Stentstruktur miteinander kombiniert werden.

Vorzugsweise ist der Stent aus Nitinol hergestellt, da Nitinol eine Superelastizität von bis zu 8% sowie Formgedächtniseigenschaften aufweist. Dabei wird die Mikrofeder durch Laserbearbeitung hergestellt. Alternativ oder zusätzlich kann der Stent jedoch auch durch ein Stanzverfahren hergestellt sein. Der Stent kann dabei vorteilhaft aus rostfreiem Edelstahl oder Kobalt-Chrom-Tantal-Legierungen hergestellt werden. Der Stent wird bevorzugt durch eine Aufweitungseinrichtung, wie z.B. einen Ballonkatheter im Körper aufgeweitet. Als Materialien eignen sich allgemein vorteilhaft Tantal, Niob oder Kobaltlegierungen. Denkbar sind aber auch Stents aus anderen Werkstoffen, wie z.B. Polymeren, selbstabbaubaren Werkstoffen (z.B. Milchsäure-Werkstoffen bzw. -Derivaten), sowie Stents aus anderen selbstexpandierbaren Werkstoffen bzw. (bevorzugt temperaturabhängigen) Formgedächniswerkstoffen.

Die Herstellung des Stents gemäß der Erfindung oder eine bevorzugte Ausführungsform hiervon kann sowohl aus Rohmaterial als auch aus Flachmaterial erfolgen, wobei bei letzterem der Stent später gerollt, geschweißt und/oder endbearbeitet wird. Weiterhin kann die Herstellung des Stents mittels Laserschneiden, Laserabtrag, photochemisches Ätzen und/oder Erodieren erfolgen. Weiterhin kann die Herstellung des Stents auch derart erfolgen, daß die Stentstruktur in einer zumindest teilweise expandierten Form gefertigt wird und der Stent anschließend zum Einführen z.B. in den Katheter auf eine komprimierte Form verkleinert wird, bevor er nachfolgend im Körper wieder zumindest teilweise expandiert wird.

### Bezugszeichenliste

- A: Axialrichtung des Stents
- U: Umfangsrichtung des Stents
- 11: Steg (flexibles Element)
- 11a: Mikrosteg
- 11b: Knotenpunkt
- 12: freies Stegende
- 13: verbundenes Stegende
- 15: Stegreihe
- 21: Zwischenelement (flexibles Element)

## Patentansprüche

1. Stent zum Einführen oder Implantieren in einen lebenden Körper mit einer Struktur, die zumindest bereichsweise Stege (11) aufweist, wobei die Stege (11) zumindest teilweise eine Federstruktur aufweisen,
**dadurch gekennzeichnet, dass**
die Federstruktur durch eine Vielzahl von Mikrostegen (11a) gebildet ist, die an Knotenpunkten (11 b) miteinander verbunden sind,
wobei die Mikrostege (11a) einen kreisförmigen oder elliptischen Querschnitt mit einem Durchmesser von 40 µm bis 200 µm oder einen rechteckigen Querschnitt mit einer Kantenlänge von 40 µm bis 200 µm aufweisen, wobei die Mikrostege (11a) eine Steglänge von 100 µm bis 2000 µm und die Stege (11) eine Länge von 1000 µm bis 10000 µm aufweisen,
wobei ein Steg (11) 8 bis 15 Mikrostege (11a) aufweist, und
wobei eine Vielzahl an Reihen (15) von Stegen (11) nebeneinander angeordnet sind und jeweils vier Stege (11) durch ein Zwischenelement (21) verbunden sind, das elastisch verformbar ist.

2. Stent nach Anspruch 1, wobei die Federstruktur zumindest teilweise durch eine Zick-Zack-Form der Mikrostege (11 a) gebildet ist.

3. Stent nach Anspruch 1 oder 2, wobei die Federstruktur zumindest teilweise durch eine Mäander- oder Wellen-Form der Mikrostege(11a) gebildet ist.

4. Stent nach einem der vorherigen Ansprüche, wobei das Zwischenelement (21) eine Zick-Zack-Form oder eine Wellenform aufweist.

5. Stent nach einem der vorherigen Ansprüche, wobei eine Reihe (15) von Stegen (11) durch zick-zack-förmig angeordnete und an jeweiligen Stegenden (12, 13) miteinander verbundene Stege (11) gebildet ist.

6. Stent nach einem der vorherigen Ansprüche, wobei die Reihen (15) der Stege (11) entlang der Umfangsrichtung (U) eines rohrförmigen Stents angeordnet sind.

7. Stent einem der vorherigen Ansprüche 1 bis 5, wobei die Reihen (15) der Stege (11) entlang der Axialrichtung (A) eines rohrförmigen Stents angeordnet sind.

8. Stent nach einem der vorherigen Ansprüche, wobei entlang einer Reihe (15) von Stegen (11) in regelmäßiger Reihenfolge freie Enden (12) der Stege (11) und durch Zwischenelemente (21) verbundene Enden (13) der Stege (11) angeordnet sind, wobei die Zwischenelemente (21) die verbundenen Enden (13) der Stege (11) mit verbundenen Enden (13) der Stege (11) der nebenliegenden Reihe (15) verbinden.

9. Stent nach Anspruch 8, wobei entlang einer Reihe (15) von Stegen (11) auf zwei freie Enden (12) der Stege (11) jeweils ein durch ein Zwischenelement (21) mit der nebenliegenden Reihe (15) von Stegen (11) verbundenes Ende (13) eines Stegs folgt.

10. Stent nach einem der vorherigen Ansprüche, wobei der Stent zumindest teilweise aus Nitinol besteht.

11. Verfahren zum Herstellen eines Stents mit:
Ausbilden einer Stentstruktur, die zumindest bereichsweise Stege aufweist; und
Ausbilden der Stege zumindest teilweise mit einer Federstruktur,
**gekennzeichnet durch** die Schritte:
Bilden der Federstruktur **durch** eine Vielzahl von Mikrostegen (11a), die an Knotenpunkten (11b) miteinander verbunden sind,
Formen der Mikrostege (11a) derart, dass diese einen kreisförmigen oder elliptischen Querschnitt mit einem Durchmesser von 40 µm bis 200 µm oder einen rechteckigen Querschnitt mit einer Kantenlänge von 40 µm bis 200 µm aufweisen, wobei die Mikrostege (11a) eine Steglänge von 100 µm bis 2000 µm und die Stege (11) eine Länge von 1000 µm bis 10000 µm aufweisen,
wobei ein Steg (11) 8 bis 15 Mikrostege (11a) aufweist, und
Anordnen einer Vielzahl an Reihen (15) von Stegen (11) nebeneinander und Verbinden von jeweils vier Stegen (11) **durch** ein Zwischenelement (21), das elastisch verformbar ist.

12. Verfahren nach Anspruch 11, des weiteren mit dem Ausbilden zumindest einer Reihe (15) von Stegen (11) durch zick-zack-förmig angeordnete und an jeweiligen Stegenden (12, 13) miteinander verbundene Stege (11).

13. Verfahren nach einem der Ansprüche 11 bis 12, wobei die Stentstruktur mit den Stegen (11) und Zwischenelementen (21) durch Laserschneiden oder Erodieren gebildet wird.

## Claims

1. A stent for insertion or implantation into a living body, comprising a structure having webs (11) at least in some area(s), the webs (11) at least partially having a spring structure,
**characterized in that**
the spring structure is formed by a plurality of microwebs (11 a) connected to each other at junction points (11b),
wherein the microwebs (11 a) have a circular or elliptical cross-section of a diameter of 40 µm to 200 µm or a rectangular cross-section of an edge length of 40 µm to 200 µm, the microwebs (11 a) having a web length of 100 µm to 2000 µm and the webs (11) having a length of 1000 µm to 10000 µm,
wherein a web (11) has 8 to 15 microwebs (11 a), and
wherein a plurality of rows (15) of webs (11) is arranged next to each other, and four webs (11) each are connected by an intermediate element (21) that is elastically deformable.

2. The stent according to claim 1, wherein the spring structure is formed at least partially by a zigzag shape of the microwebs (11 a).

3. The stent according to claim 1 or 2, wherein the spring structure is formed at least partially by a meander or wave shape of the microwebs (11 a).

4. The stent according to one of the preceding claims, wherein the intermediate element (21) has a zigzag shape or a wave shape.

5. The stent according to one of the preceding claims, wherein a row (15) of webs (11) is formed by webs (11) arranged in a zigzag shape and connected to each other at respective web ends (12, 13).

6. The stent according to one of the preceding claims, wherein the rows (15) of webs (11) are arranged along the circumferential direction (U) of a tubular stent.

7. The stent according to one of the preceding claims 1 to 5, wherein the rows (15) of webs (11) are arranged along the axial direction (A) of a tubular stent.

8. The stent according to one of the preceding claims, wherein along a row (15) of webs (11), free ends (12) of the webs (11) and ends (13) of the webs (11) connected by intermediate elements (21) are arranged in a regular order, the intermediate elements (21) connecting the connected ends (13) of the webs (11) to connected ends (13) of the webs (11) of the adjacent row (15).

9. The stent according to claim 8, wherein along a row (15) of webs (11), two free ends (12) of the web (11) are followed by respectively one end (13) of a web connected to the adjacent row (15) of webs (11) by an intermediate element (21).

10. The stent according to one of the preceding claims, wherein the stent is at least partially composed of nitinol.

11. A method for producing a stent, comprising:
forming a stent structure having webs at least in some area(s);
and forming the webs at least partially with a spring structure,
**characterized by** the steps of:
forming the spring structure by a plurality of microwebs (11a) connected to each other at junction points (11 b),
forming the microwebs (11a) to have a circular or elliptical cross-section of a diameter of 40 µm to 200 µm or a rectangular cross-section of an edge length of 40 µm to 200 µm, the microwebs (11a) having a web length of 100 µm to 2000 µm and the webs (11) having a length of 1000 µm to 10000 µm,
wherein a web (11) has 8 to 15 microwebs (11a), and
arranging a plurality of rows (15) of webs (11) next to each other, and connecting four webs (11) each by an intermediate element (21) that is elastically deformable.

12. The method according to claim 11, further comprising forming at least a row (15) of webs (11) by webs (11) arranged in a zigzag shape and connected to each other at respective web ends (12, 13).

13. The method according to one of claims 11 to 12, wherein the stent structure with the webs (11) and intermediate elements (21) is formed by laser cutting or eroding.

## Revendications

1. Endoprothèse vasculaire pour l'introduction ou l'implantation dans un corps vivant avec une structure qui présente des nervures (11) au moins par région, dans laquelle les nervures (11) présentent au moins partiellement une structure à ressort,
**caractérisée en ce que**
la structure à ressort est formée par une pluralité de micronervures (11a) qui sont reliées ensemble au niveau de points de jonction (11b),
dans laquelle les micronervures (11a) présentent une section transversale circulaire ou elliptique avec un diamètre de 40 µm à 200 µm ou une section transversale rectangulaire avec une longueur d'arête de 40 µm à 200 µm, dans laquelle les micronervures (11a) présentent une longueur de nervure de 100 µm à 2000 µm et les nervures (11) présentent une longueur de 1000 µm à 10000 µm,
dans laquelle une nervure (11) présente 8 à 15 micronervures (11a), et
dans laquelle une pluralité de rangées (15) de nervures (11) sont disposées côte à côte et quatre nervures (11) sont à chaque fois reliées par un élément intermédiaire (21) qui est déformable de manière élastique.

2. Endoprothèse vasculaire selon la revendication 1, dans laquelle la structure à ressort est formée au moins partiellement par une forme en zigzag des micronervures (11a).

3. Endoprothèse vasculaire selon la revendication 1 ou 2, dans laquelle la structure à ressort est formée au moins partiellement par une forme de méandres ou de vagues des micronervures (11a).

4. Endoprothèse vasculaire selon l'une quelconque des revendications précédentes, dans laquelle l'élément intermédiaire (21) présente une forme en zigzag ou une forme de vagues.

5. Endoprothèse vasculaire selon l'une quelconque des revendications précédentes, dans laquelle une rangée (15) de nervures (11) est formée par des nervures (11) disposées en forme de zigzag et reliées ensemble à des extrémités de nervure respectives (12, 13).

6. Endoprothèse vasculaire selon l'une quelconque des revendications précédentes, dans laquelle les rangées (15) de nervures (11) sont disposées le long du sens périphérique (U) d'une endoprothèse vasculaire tubulaire.

7. Endoprothèse vasculaire selon l'une quelconque des revendications précédentes 1 à 5, dans laquelle les rangées (15) de nervures (11) sont disposées le long de la direction axiale (A) d'une endoprothèse vasculaire tubulaire.

8. Endoprothèse vasculaire selon l'une quelconque des revendications précédentes, dans laquelle des extrémités libres (12) des nervures (11) et des extrémités (13) reliées par des éléments intermédiaires (21) des nervures (11) sont disposées le long d'une rangée de nervures (11) en succession régulière, dans laquelle les éléments intermédiaires (21) relient les extrémités reliées (13) des nervures (11) à des extrémités reliées (13) des nervures (11) de la rangée juxtaposée (15).

9. Endoprothèse vasculaire selon la revendication 8, dans laquelle une extrémité (13) d'une nervure reliée par un élément intermédiaire (21) à la rangée juxtaposée (15) de nervures (11) fait à chaque fois suite à deux extrémités libres (12) des nervures (11) le long d'une rangée (15) de nervures (11).

10. Endoprothèse vasculaire selon l'une quelconque des revendications précédentes, dans laquelle l'endoprothèse vasculaire se compose au moins partiellement de nitinol.

11. Procédé de fabrication d'une endoprothèse vasculaire avec :
réalisation d'une structure d'endoprothèse vasculaire qui présente des nervures au moins par région ; et
réalisation des nervures au moins partiellement avec une structure à ressort,
**caractérisé par** les étapes consistant à :
former la structure à ressort par une pluralité de micronervures (11a) qui sont reliées ensemble au niveau de points de jonction (11b),
mouler les micronervures (11a) de telle sorte que celles-ci présentent une section transversale circulaire ou elliptique avec un diamètre de 40 µm à 200 µm ou une section transversale rectangulaire avec une longueur d'arête de 40 µm à 200 µm,
dans lequel les micronervures (11a) présentent une longueur de nervure de 100 µm à 2000 µm et les nervures (11) présentent une longueur de 1000 µm à 10000 µm,
dans lequel une nervure (11) présente 8 à 15 micronervures (11a), et
disposer une pluralité de rangées (15) de nervures (11) côte à côte et relier quatre nervures respectives (11) par un élément intermédiaire (21) qui est déformable de manière élastique.

12. Procédé selon la revendication 11, en outre avec la réalisation d'au moins une rangée (15) de nervures (11) par des nervures (11) disposées en forme de zigzag et reliées ensemble à des extrémités de nervure respectives (12, 13).

13. Procédé selon l'une quelconque des revendications 11 à 12, dans lequel la structure d'endoprothèse vasculaire avec les nervures (11) et éléments intermédiaires (21) est formée par découpe laser ou érosion.
